# FASCICULE DE BREVET EUROPEEN

(11) **EP 2 727 581 B1**
(45) Date de publication et mention de la délivrance du brevet: **15.11.2017**
(21) Numéro de dépôt: 13198504.6
(22) Date de dépôt: 06.07.2010
(51) Int. Cl.: A61K 8/67, A61K 8/73, A61Q 19/08, A61K 31/728, A61L 27/50, A61K 9/00, A61P 17/00, A61K 45/06, A61L 27/52

(54) **COMPOSITION INJECTABLE ASSOCIANT UN AGENT DE COMBLEMENT ET UN MILIEU DE CROISSANCE DES FIBROBLASTES**
INJIZIERBARE ZUSAMMENSETZUNG MIT EINER KOMBINATION AUS EINER FÜLLSUBSTANZ UND EINEM FIBROBLASTENWACHSTUMSMEDIUM
INJECTABLE COMPOSITION COMBINING A FILLING AGENT AND A FIBROBLAST GROWTH MEDIUM

(30) Priorité: 27.07.2009 FR 0955235
(43) Date de publication de la demande: 07.05.2014
(62) Demande divisionnaire de: 10742206.5
(73) Titulaire: Thorel, Jean-Noël, 75014 Paris (FR)
(72) Inventeur: Thorel, Jean-Noël, 75014 Paris (FR); Gatto, Hugues, 06570 Saint Paul de Vence (FR)
(74) Mandataire: Cabinet Laurent & Charras

(56) Documents cités:
- US-A1- 2003 180 270
- US-A1- 2005 260 753
- US-A1- 2007 237 750
- US-A1- 2009 181 007
- US-A1- 2009 202 654
- US-A1- 2010 120 145

## Description

La présente invention s'inscrit dans le développement de solutions injectables pour le traitement des rides.

Elle propose d'associer un agent de comblement classique, tel que l'acide hyaluronique, avec un milieu de croissance des fibroblastes de composition bien déterminée permettant une revitalisation du derme.

### Etat antérieur de la technique

La peau est un tissu en renouvellement continu, comprenant une pluralité de cellules et des structures spécialisées. Etant donné son contact permanent avec l'environnement, la peau représente une barrière protectrice pour le corps. De plus, elle est impliquée dans de nombreux processus physiologiques permettant au corps de maintenir une température fixe et constante. Par ailleurs, la peau joue un rôle important dans le système immunitaire qui protège le corps des maladies.

Au niveau structurel, la peau comprend trois couches :
- une couche externe, appelée épiderme,
- une couche interne, appelée tissu sous-cutané,
- et une couche intermédiaire, couramment nommée derme.

L'épiderme humain naturel est composé principalement de trois types de cellules, à savoir les kératinocytes très majoritaires, les mélanocytes et les cellules de Langerhans. L'épiderme, en qualité de couche externe, agit comme une barrière à l'encontre des agents externes.

L'épiderme comprend lui-même 5 couches distinctes, de la profondeur à la surface:
- la couche basale ou stratum germinativum,
- la couche de Malpighi ou stratum spinosum,
- la couche granuleuse ou stratum granulosum,
- la couche claire ou stratum lucidum,
- et la couche cornée ou stratum corneum.

Le derme, quant à lui, fournit à l'épiderme un support solide et joue le rôle d'élément nourricier vis-à-vis de celui-ci. Le derme est essentiellement constitué de fibroblastes et d'une matrice extracellulaire (MEC), composée elle-même principalement de collagène, d'élastine et d'une substance dite "substance fondamentale". L'ensemble de ces composants est synthétisé par les fibroblastes. On trouve également dans le derme des leucocytes, des hématocytes ou encore des macrophages tissulaires. En outre, celui-ci est traversé par des vaisseaux sanguins et des fibres nerveuses.

Enfin, le tissu sous-cutané ou hypoderme est une couche de tissu adipeux et connectif qui couvre les nerfs et les gros vaisseaux sanguins.

Au cours du processus de vieillissement, il apparaît différents signes caractéristiques sur la peau, traduisant une modification de la structure et des fonctions cutanées. Les principaux signes du vieillissement cutané sont notamment l'apparition de ridules et/ou de rides, en augmentation avec l'âge. Ces rides peuvent être profondes, moyennes ou superficielles et touchent en particulier les sillons naso-géniens, la zone périorbitale, le contour des lèvres, le front, la zone intersourcilière (rides du lion). Ces rides et ridules se traduisent par une dépression ou des sillons à la surface de la peau.

Les rides profondes seraient dues à des modifications dermo-hypodermiques, tandis que les rides superficielles pourraient s'expliquer par des modifications dermiques et éventuellement épidermiques.

Les rides sont souvent dues à la perte d'élasticité de la peau, en particulier un assouplissement des tissus, ainsi que la production de ridules de différentes épaisseurs. Lorsque le derme perd de son élasticité, il s'affaiblit et commence à former des rides plus profondes. Pendant la production de rides, les fibres de collagène, responsables de l'élasticité et de la structure de la peau, perdent leurs caractéristiques, avec une surproduction d'enzymes de type métalloprotéinases. Cette quantité anormale d'enzymes dégrade la matrice de collagène et conduit ainsi à la production de rides profondes. Ainsi, au fil des années, le derme tend à devenir plus mince, en particulier au niveau de sa couche de collagène.

D'autres facteurs, tels que les radicaux libres, l'exposition au soleil, la pollution, le tabagisme, la consommation d'alcool ou l'ozone, peuvent causer des dommages à la peau, par le même phénomène d'activation des métalloprotéinases et de décomposition du collagène.

Au cours de ces dernières années, le traitement des modifications cutanées inesthétiques, notamment liées au vieillissement, a vu un essor important et a fait d'énormes progrès.

Ainsi, il a été proposé différents traitements, notamment l'injection de substances naturelles ou synthétiques, afin de remédier aux altérations cutanées.

On peut noter en particulier l'application, sous forme d'injections locales, de la toxine botulique désactivée (Botox®) ou la mise en oeuvre de techniques laser, voire leur utilisation en combinaison.

Une alternative à ces solutions techniques est l'injection dans le derme de produits de comblement, dits "dermal fillers". Ce comblement peut être réalisé à l'aide de produits non résorbables, tels que des gels de polyacrylamide ou des particules de polyméthylméthacrylate (PMMA). Toutefois, ces composants peuvent entraîner des réactions d'intolérance du type inflammation ou hypersensibilité.

Pour ces raisons, l'utilisation de produits résorbables, tels que les protéines ou les graisses, a été envisagée. A ce jour, la solution technique privilégiée est la mise en oeuvre de substances présentes à l'état naturel dans le corps humain, telles que le collagène ou l'acide hyaluronique, qui sont à l'origine de la majorité des produits actuellement disponibles sur le marché.

Ces produits résorbables présentent toutefois l'inconvénient d'être dégradés assez rapidement dans l'organisme, ce qui réduit leur efficacité et nécessite des injections répétées et régulières.

A titre d'exemple particulier de produit naturel résorbable, l'acide hyaluronique est un composé privilégié.

Il est à mentionner que l'acide hyaluronique (HA) est un constituant naturel du derme, où il joue un rôle important dans l'hydratation et l'élasticité de la peau. Il diminue cependant en quantité et en qualité avec l'âge, entraînant un dessèchement et un amincissement de la peau qui se ride. Dans la mesure où l'acide hyaluronique est en outre très hydrosoluble et forme des solutions à haute viscosité dans l'eau, il fait partie des produits pharmaceutiques les plus utilisés.

A ce jour, l'acide hyaluronique mis en oeuvre dans les produits pharmaceutiques ou les dispositifs médicaux destinés au traitement des rides se présente sous la forme d'un gel de hyaluronate de sodium ou de potassium. Toutefois, ces gels de hyaluronate de sodium ou de potassium présentent une biorésorbabilité assez rapide (variant typiquement entre 4 et 6 mois), qui nécessite que les injections soient répétées à intervalles proches et réguliers.

Pour tenter d'augmenter la durée d'action de l'acide hyaluronique, des formes stabilisées de HA ont été développées. Il s'agit en particulier de gels de HA réticulés chimiquement. Cette réticulation, via des pontages intra ou intermoléculaires, permettrait d'augmenter la persistance du produit à l'intérieur du derme. De manière alternative, il a été envisagé d'encapsuler l'acide hyaluronique (WO2008/147817).

Par ailleurs, les derniers développements concernant les produits de comblement se sont concentrés sur la combinaison de différents principes actifs dans le cadre de cette application.

Ainsi, il a été envisagé d'associer l'acide hyaluronique, agissant par effet mécanique de comblement, à l'administration cutanée d'autres substances actives dans ce contexte. A titre d'exemple, le document WO2008/139122 associe du HA à un inhibiteur de la dégradation de l'acide hyaluronique agissant *in vivo* pour assurer une certaine préservation des molécules d'HA injectées.

Le document US2009/202654 décrit une composition destinée au rajeunissement de la peau. La croissance de cellules de la peau est réalisée dans un milieu de culture puis le milieu ainsi conditionné, comprenant des facteurs de croissance, est récupéré pour sa formulation dans ladite composition.

Il apparaît toutefois que, malgré les différentes alternatives disponibles sur le marché, il existe toujours le besoin de développer des solutions techniques permettant d'assurer une réparation cutanée efficace, perdurant dans le temps et étant aussi indolore que possible pour la peau.

### Description de l'invention

Dans ce cadre, le Demandeur s'est inscrit dans une démarche totalement nouvelle.

Alors que l'art antérieur préconisait d'associer un agent de comblement, tel que l'acide hyaluronique, avec un agent protecteur de celui-ci, la présente invention propose d'agir à deux niveaux distincts pour rétablir une bonne apparence de la peau, notamment pour lutter contre le vieillissement.

Ainsi, il est rapporté une composition (dermatologique, cosmétique ou thérapeutique) injectable, associant à la fois un agent de comblement dit mécanique, connu en tant que tel, et d'autre part, un milieu de croissance des fibroblastes.

En pratique, il s'agit donc à la fois d'agir physiquement pour combler les aspérités ou sillons que constituent les rides et d'autre part de stimuler la croissance des fibroblastes au niveau du derme. Les fibroblastes peuvent également croître à l'intérieur des sillons et de surcroît synthétisent des substances telles que l'élastine et les collagènes qui favorisent la régénération du derme. En outre, le milieu de croissance est susceptible de stabiliser et de protéger l'agent de comblement en présence.

Un premier aspect de la présente invention concerne une composition injectable par voie sous-cutanée ou intradermique constituée de :
l'acide hyaluronique ou un de ses sels en tant qu'agent de comblement, l'acide hyaluronique étant sous forme non réticulée; et
un milieu de croissance des fibroblastes exempt de tout facteur de croissance cellulaire ou de tout extrait biologique d'origine animale ou cellulaire, non tracés et/ou de composition indéterminée, ledit milieu de croissance des fibroblastes comprenant :
   - des constituants d'acides nucléiques,
   - des acides aminés,
   - des sucres simples et complexes,
   - des vitamines,
   - et une fraction inorganique contenant des oligoéléments et des sels minéraux.

Le premier composant de la combinaison selon l'invention est donc un agent de comblement mécanique qui a pour fonction essentielle de créer un volume à l'intérieur des rides.

Dans la recherche d'une solution technique aussi neutre que possible pour la peau, dite « biomimétique », l'acide hyaluronique non réticulé, ainsi que ses sels physiologiquement acceptables, est mis en oeuvre dans la mesure où cette molécule est un constituant naturel du derme. On désigne par sels d'acide hyaluronique physiologiquement acceptables, notamment les sels de sodium et de potassium, ainsi que leur mélange.

De manière avantageuse, l'acide hyaluronique représente de 0,07 à 3% de la masse totale de la composition, avantageusement de 0,8 à 2,5%.

Il est à noter que le poids moléculaire de l'acide hyaluronique choisi peut dépendre de l'application visée, notamment de la profondeur des rides à traiter.

Le deuxième constituant nécessaire de la composition selon l'invention est donc un milieu de croissance des fibroblastes.

Dans le cadre de la présente invention, un milieu de croissance des fibroblastes est défini comme un milieu complet permettant non seulement le maintien des fibroblastes en vie, mais stimulant également leur multiplication. La mise en oeuvre d'un test fonctionnel de croissance permet de déterminer si un milieu donné correspond à un milieu de croissance des fibroblastes. En particulier, un test fonctionnel adapté bien connu de l'homme du métier est le test d'observation de la densité des cellules vivantes par méthode colorimétrique, mettant en oeuvre le réactif WST-1 et une lecture à 450 nm (Berridge, M. V. et al. (1996): The Biochemical and Cellular Basis of Cell Prolifération Assays That Use Tetrazolium Salts. Biochemica 4, 15-19).

Un milieu de croissance des fibroblastes est par exemple disponible commercialement : il s'agit du milieu de culture standard DMEM (Sigma) supplémenté en facteur de croissance cellulaire SVF (sérum de veau foetal), à hauteur de 10% en masse.

De manière générale, de tels milieux contiennent des extraits d'origine animale ou cellulaire qui ont effectivement un effet stimulateur sur la croissance des fibroblastes, mais qui présentent l'inconvénient de ne pas avoir une composition déterminée ou de contenir des éléments exogènes non traçables tels que le SVF, les extraits de tige pituitaire de boeuf, les facteurs de croissance cellulaires EGF (« epidermal growth factor »), FGF (« fibroblast growth factors »), l'insuline ou la choléra toxine, l'hydrocortisone, la pipérazine, ...

Le milieu de croissance des fibroblastes mis en oeuvre dans le cadre de la présente invention ne contient pas de facteur de croissance cellulaire ou d'extrait biologique d'origine animale ou cellulaire, si ceux-ci ne sont pas tracés et/ou ne sont pas de composition déterminée.

L'expression «non tracé » signifie que la source de la matière biologique en question et/ou le traitement subi par celle-ci ne peuvent pas être établis ou contrôlés.

En d'autres termes, ledit milieu est dépourvu (exempt) d'extrait biologique d'origine animal ou cellulaire, de composé ou facteur de croissance cellulaire, ou d'une hormone.

Par ailleurs, il s'agit d'introduire par injection dans le derme un milieu de croissance de fibroblastes aussi compatible que possible avec la composition naturelle de la peau, à savoir un milieu comprenant des constituants biodermiques (contenus à l'état naturel dans la peau), biomimétiques et/ou biocompatibles (éléments biologiques mimétiques ou neutres pour la peau).

Ainsi et selon une autre caractéristique, un tel milieu permet de fournir spécifiquement aux fibroblastes un apport nutritionnel optimisé et comprend des vitamines, des oligoéléments, des acides aminés, des sels minéraux, des sucres simples (tels que glucose, ribose, déoxyribose) et/ou complexes (tels que HA), et des constituants d'acides nucléiques (bases azotées et pentoses nécessaires à la formation des nucléotides, ainsi que nucléosides). Il présente en outre avantageusement des caractéristiques de pH (entre 6,5 et 7,9, avantageusement entre 7,4 et 7,6 et d'osmolarité (entre 280 et 450 mOsm, avantageusement entre 300 et 350 mOsm) physiologiques.

A noter que l'HA peut être à la fois un ingrédient du milieu de croissance et un agent de comblement. La différence réside à la fois dans la forme de l'HA (nécessairement un sel de hyaluronate dans le milieu) et par sa quantité (quantités beaucoup plus faibles dans le milieu).

Selon un mode de réalisation particulier, l'ensemble des constituants du milieu sont présents naturellement dans la peau (constituants dermiques). Toutefois et pour stimuler la croissance des fibroblastes, un tel milieu peut être enrichi à l'aide d'une substance exogène à la peau mais d'origine naturelle, traçable et de composition bien déterminée. Une substance répondant à cette définition est par exemple un mélange de peptides extraits du lait, ou complexe MPC (« Milk Peptide Complex »), obtenu par précipitations successives du lait puis séparation de certaines protéines soumises à hydrolyse enzymatique. Cette substance se présentant sous forme d'une poudre déshydratée est avantageusement ajoutée au milieu à hauteur de 0,5 à 5 mg/ml, encore plus avantageusement de 4 à 5 mg/ml.

Selon un autre mode de réalisation privilégié, le milieu de croissance des fibroblastes mis en oeuvre dans la composition selon l'invention est dépourvu d'EDTA et ses sels et/ou d'acide lipoïque comme agents inhibiteurs des métalloprotéinases.

A titre d'exemple, un milieu complexe répondant à une telle définition a été développé par le Demandeur et correspond à l'association d'une soixantaine de composants en quantités précisément définies comme suit :

| DENOMINATION INTERNATIONALE NOMENCLATURE COSMETIQUE INGREDIENT (INCI) | *CONCENTRATION FINALE* Solution 1 X (en mg/l) |
|---|---|
| EAU | q.s.p. 1 litre |
| CHLORURE DE SODIUM | 5000 à 8000 |
| L-GLUTAMINE ou L-ALANYL-GLUTAMINE | 100 à 3000 |
| SODIUM BICARBONATE | 0 à 2000 |
| D-GLUCOSE | 2000 à 5000 |
| L-ARGININE HCL | 300 à 500 |
| SODIUM ACETATE | 200 à 450 |
| DISODIUM PHOSPHATE Na₂HPO4 | 100 à 1500 |
| L-LEUCINE | 50 à 200 |
| L-SERINE | 50 à 200 |
| CHLORURE DE MAGNESIUM MgCl₂, 6H₂O | 50 à 200 |
| CHLORURE DE POTASSIUM | 50 à 200 |
| L-VALINE | 20 à 150 |
| SODIUM PYRUVATE | 10 à 75 |
| L-LYSINE HCL | 10 à 75 |
| L-HISTIDINE HCL,H₂O | 10 à 75 |
| L-CYSTEINE HCL H₂O | 10 à 75 |
| ADENINE (HCl) | 5 à 50 |
| L-THREONINE | 5 à 50 |
| CHLORURE DE CALCIUM CaCl₂, 2H₂O | 0 à 22,5 |
| MYO- INOSITOL | 5 à 50 |
| ACIDE L-GLUTAMIQUE | 15 à 75 |
| L-ASPARAGINE H₂O | 15 à 75 |
| L-METHIONINE | 10 à 50 |
| L-TYROSINE 2Na₂ 2H₂O | 10 à 50 |
| L-PHENYLALANINE | 2 à 20 |
| L-TRYPTOPHANE | 2 à 20 |
| L-ALANINE | 5 à 30 |
| GLYCINE | 5 à 30 |
| L-ISOLEUCINE | 5 à 30 |
| ACIDE L-ASPARTIQUE | 10 à 50 |
| SODIUM SULFATE | 1 à 10 |
| SULFATE FERREUX FeSO₄, 7H₂O | 1 à 10 |
| ACIDE FOLIQUE | 1 à 5 |
| THYMIDINE | 0,1 à 3 |
| CYANOCOBALAMINE | 0,1 à 3 |
| D-CALCIUM PANTOTHENATE | 1 à 5 |
| THIAMINE HCL | 1 à 5 |
| ACIDE THIOCTIQUE | 0,1 à 1 |
| ZINC SULFATE ZnSO₄, 7H₂O | 0,05 à 0,5 |
| SODIUM SILICATE NA₂SIO₃, 4H₂O | 0,05 à 0,5 |
| PYRIDOXINE HCL | 0,5 à 3 |
| NIACINAMIDE (NICOTINAMIDE) | 0,5 à 3 |
| RIBOFLAVINE | 0,05 à 0,5 |
| d-BIOTIN | 0,01 à 0,05 |
| SULFATE DE CUIVRE CuSO₄, 5H₂O | 0 à 0,005 |
| AMMONIUM MOLYBDATE (NH4)₆MO7O₂₄,4H₂O | 0 à 0,005 |
| AMMONIUM VANADATE NH₄VO₃ | 0 à 0,001 |
| CHLORURE DE MANGANESE MnCl₂, 4H₂O | 0 à 0,0001 |
| HYALURONATE DE SODIUM | 100 à 1000 |
| L-PROLINE | 10 à 100 |
| HYDROXYPROLINE | 10 à 100 |
| ACIDE ASCORBIQUE | 0,1 à 10 |
| ADENOSINE | 0,01 à 1 |
| GUANINE | 0,01 à 1 |
| DEOXYRIBOSE | 0,01 à 1 |
| RIBOSE | 0,01 à 1 |
| CHOLINE CHLORIDE | 0 à 3 |
| MPC | 0 à 5000 |

Comme déjà dit, cette composition a pour vocation d'être injectée et est donc assimilable à un implant injectable. Un tel implant est donc destiné à être injecté dans le derme superficiel, moyen ou profond, de manière sous-cutanée ou intradermique, avantageusement au niveau du visage.

Selon un mode de réalisation avantageux, la composition se présente sous la forme d'un gel, en raison de l'application injectable faisant l'objet de l'invention. De manière remarquable, cette contrainte est tout à fait compatible avec les milieux de croissance des fibroblastes décrits ci-dessus, qui sont formulables sous forme de gel, grâce à l'incorporation d'HA, sans ajout d'excipients exogènes.

Encore plus avantageusement, la composition se présente sous la forme d'un hydrogel monophasique, à savoir un hydrogel sous forme d'une seule phase homogène. La viscosité de la composition obtenue peut être aisément ajustée, notamment en adaptant la composition et la quantité de l'agent de comblement. Dans le cas de l'acide hyaluronique, on peut jouer à la fois sur la concentration qui varie typiquement entre 0,07% et 3% en poids de la composition, mais également au niveau de son poids moléculaire.

La composition injectable selon l'invention peut également faire l'objet d'un kit qui comprend en outre des seringues pouvant contenir ladite composition. Il s'agit par exemple de seringues unidoses de 0,5 à 1,5 ml. Dans un tel kit, les 2 constituants essentiels de la composition peuvent être présentés en tant que mélange dans une même seringue, ou dans 2 seringues distinctes pour un mélange extemporané.

Etant donné la visée thérapeutique, une telle composition est avantageusement soumise à stérilisation, stérilisation ayant lieu avantageusement à froid de manière à ne pas dénaturer les composants en présence. Cette étape peut par exemple être réalisée par filtration sur membrane à 0,22 µm pour le milieu de croissance des fibroblastes, et par stérilisation séparée selon un processus connu de l'homme de l'art pour l'HA.

Etant donné leur mode d'action complémentaire, les deux constituants de la composition selon l'invention peuvent être administrés de manière simultanée, séparée ou étalée dans le temps.

Comme déjà dit, une composition selon l'invention est destinée à corriger toutes les irrégularités cutanées, et en particulier au traitement, à l'amélioration et/ou la prévention du vieillissement cutané. Sont ainsi visés les rides, les ridules, les dépressions fibroblastiques et les cicatrices, notamment au niveau des zones du visage ou du front marquées par les rides d'expression.

Une composition selon l'invention concerne aussi bien la dermatologie que la chirurgie réparatrice.

Ainsi, la présente invention vise une composition telle que décrite ci-dessus pour son utilisation dans le comblement des dépressions cutanées ou des cicatrices. Selon un autre aspect, elle vise un procédé cosmétique de comblement des rides et/ou ridules comprenant l'injection de ladite composition.

A noter que le mélange des deux constituants essentiels de cette composition peut se faire de manière extemporanée. De même, leur injection peut avoir lieu de manière non simultanée.

Ainsi et selon un autre aspect, la présente invention vise une composition telle que décrite ci-dessus comme produit de combinaison pour une utilisation simultanée, séparée ou étalée dans le temps, pour la prévention ou le traitement des rides, ridules, dépressions cutanée et/ou cicatrices.

Grâce à la composition particulière selon la présente invention, deux actions physiologiques complémentaires sont donc visées et obtenues : d'une part une action mécanique de comblement des irrégularités, mais également l'action de favoriser le renouvellement cellulaire et de fait, la synthèse des constituants néo-synthétisés par les fibroblastes, notamment les collagènes et l'élastine. Il en résulte donc un remodelage de la matrice extracellulaire et une revitalisation du derme.

Il existe ainsi un phénomène de comblement mécanique immédiat à partir de l'injection et un effet, à terme, de régénération cellulaire. Il peut donc être envisagé de diminuer les concentrations en agent de comblement au cours du traitement. En effet, une fois que la croissance des fibroblastes prend le relais, l'agent de comblement mécanique devient moins nécessaire et en pratique, les quantités en agent de comblement peuvent être diminuées.

Par ailleurs, une telle composition est totalement biocompatible avec la peau, dans la mesure où elle est essentiellement constituée de constituants naturellement présents au niveau du derme. De fait, celle-ci ne perturbe pas le micro environnement de la peau, réduisant ainsi les risques de réactions inflammatoires ou allergiques. Par ailleurs, il a été montré que de tels milieux biomimétiques et biocompatibles permettaient une croissance des fibroblastes stimulée en présence de sérum. Ils constituent donc des candidats particulièrement adaptés dans le contexte d'une injection dermique, dans la mesure où le derme est richement vascularisé.

L'invention va maintenant être listée de manière non limitative par les exemples suivants, à l'appui des figures annexées.

### Légendes des figures

La figure 1 illustre la croissance comparée de fibroblastes humains en culture dans un milieu de croissance des fibroblastes selon l'invention et le milieu standard DMEM (sigma), sans facteur de croissance.
La figure 2 représente les taux de collagène dans le derme superficiel et moyen de peaux saines, altérées par rayonnement UV et après traitement par un milieu de croissance des fibroblastes selon l'invention.
La figure 3 correspond à des coupes histologiques montrant le marquage des fibres de collagène dans une peau saine, altérée par irradiation et après traitement par un milieu de croissance des fibroblastes selon l'invention.
La figure 4 illustre le dosage de l'élastine dans les peaux saines, puis altérées par rayonnement UV et ensuite après traitement par le milieu de croissance des fibroblastes selon l'invention.
La figure 5 correspond à des coupes histologiques montrant le marquage des fibres d'élastine dans une peau saine, une peau altérée par irradiation puis traitée par le milieu de croissance des fibroblastes selon l'invention.
La figure 6 représente les taux de GAGs dans le derme superficiel et moyen de peaux saines, de peaux altérées par rayonnement UV et après traitement par le milieu de croissance des fibroblastes selon l'invention.
La figure 7 correspond à des coupes histologiques montrant le marquage des GAGS dans une peau saine, une peau altérée par irradiation UV et après traitement par le milieu de croissance des fibroblastes selon l'invention.

### Exemples de réalisation

### 1/ Utilisation d'un milieu de croissance des fibroblastes dans une composition injectable :

### a) Composition du milieu :

| DENOMINATION INTERNATIONALE NOMENCLATURE COSMETIQUE INGREDIENT (INCI) | *CONCENTRATION FINALE* Solution 1 X (en mg/l) |
|---|---|
| EAU | q.s.p. 1 litre |
| CHLORURE DE SODIUM | 5000 à 8000 |
| L-GLUTAMINE ou L-ALANYL-GLUTAMINE | 100 à 3000 |
| SODIUM BICARBONATE | 0 à 2000 |
| D-GLUCOSE | 2000 à 5000 |
| L-ARGININE HCL | 300 à 500 |
| SODIUM ACETATE | 200 à 450 |
| DISODIUM PHOSPHATE Na₂HPO4 | 100 à 1500 |
| L-LEUCINE | 50 à 200 |
| L-SERINE | 50 à 200 |
| CHLORURE DE MAGNESIUM MgCl₂, 6H₂O | 50 à 200 |
| CHLORURE DE POTASSIUM | 50 à 200 |
| L-VALINE | 20 à 150 |
| SODIUM PYRUVATE | 10 à 75 |
| L-LYSINE HCL | 10 à 75 |
| L-HISTIDINE HCL,H₂O | 10 à 75 |
| L-CYSTEINE HCL H₂O | 10 à 75 |
| ADENINE (HCl) | 5 à 50 |
| L-THREONINE | 5 à 50 |
| CHLORURE DE CALCIUM CaCl₂, 2H₂O | 0 à 22,5 |
| MYO- INOSITOL | 5 à 50 |
| ACIDE L-GLUTAMIQUE | 15 à 75 |
| L-ASPARAGINE H₂O | 15 à 75 |
| L-METHIONINE | 10 à 50 |
| L-TYROSINE 2Na₂ 2H₂O | 10 à 50 |
| L-PHENYLALANINE | 2 à 20 |
| L-TRYPTOPHANE | 2 à 20 |
| L-ALANINE | 5 à 30 |
| GLYCINE | 5 à 30 |
| L-ISOLEUCINE | 5 à 30 |
| ACIDE L-ASPARTIQUE | 10 à 50 |
| SODIUM SULFATE | 1 à 10 |
| SULFATE FERREUX FeSO₄,7H₂O | 1 à 10 |
| ACIDE FOLIQUE | 1 à 5 |
| THYMIDINE | 0,1 à 3 |
| CYANOCOBALAMINE | 0,1 à 3 |
| D-CALCIUM PANTOTHENATE | 1 à 5 |
| THIAMINE HCL | 1 à 5 |
| ACIDE THIOCTIQUE | 0,1 à 1 |
| ZINC SULFATE ZnSO₄,7H₂O | 0,05 à 0,5 |
| SODIUM SILICATE NA₂SIO₃,4H₂O | 0,05 à 0,5 |
| PYRIDOXINE HCL | 0,5 à 3 |
| NIACINAMIDE (NICOTINAMIDE) | 0,5 à 3 |
| RIBOFLAVINE | 0,05 à 0,5 |
| d-BIOTIN | 0,01 à 0,05 |
| SULFATE DE CUIVRE CuSO₄,5H₂O | 0 à 0,005 |
| AMMONIUM MOLYBDATE (NH4)₆MO7O₂₄,4H₂O | 0 à 0,005 |
| AMMONIUM VANADATE NH₄VO₃ | 0 à 0,001 |
| CHLORURE DE MANGANESE MnCl₂, 4H₂O | 0 à 0,0001 |
| HYALURONATE DE SODIUM | 100 à 1000 |
| L-PROLINE | 10 à 100 |
| HYDROXYPROLINE | 10 à 100 |
| ACIDE ASCORBIQUE | 0,1 à 10 |
| ADENOSINE | 0,01 à 1 |
| GUANINE | 0,01 à 1 |
| DEOXYRIBOSE | 0,01 à 1 |
| RIBOSE | 0,01 à 1 |
| CHOLINE CHLORIDE | 0 à 3 |
| MPC | 0 à 5000 |

### b) Culture de fibroblastes humains

### Protocole

- Fibroblastes humains ensemencés à faible densité en plaques de 96 puits sous milieu de culture standard DMEM, supplémenté en facteur de croissance cellulaire SVF (Sérum de Veau Foetal) ;
- Après 24h, culture dans le milieu selon l'invention pur ou dans le milieu standard DMEM sans facteur de croissance ;
- Absence de renouvellement des milieux au cours de l'expérience ;
- Densité des cellules vivantes évaluée à T0 puis après 2, 4, 7 et 9 jours, par méthode colorimétrique (réactif WST-1).

### Résultats

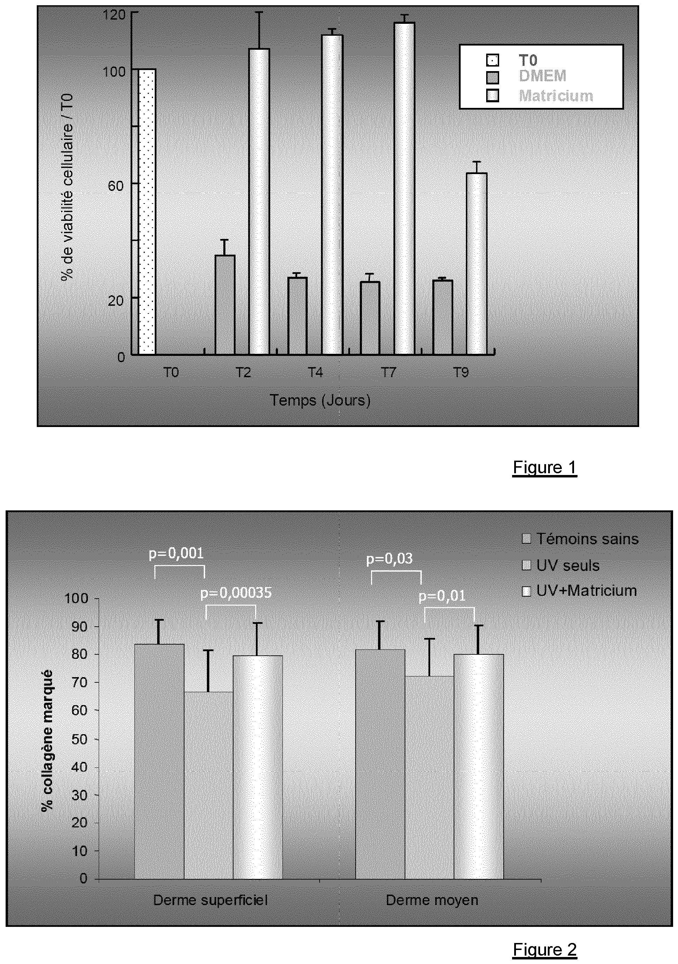

- Le milieu de culture selon l'invention permet de soutenir, seul, la croissance des fibroblastes sur une durée de 9 jours. On observe à partir du 7ème jour un ralentissement de la croissance cellulaire s'expliquant par le non renouvellement du milieu (Fig. 1).
- En milieu DMEM sans SVF, on observe une diminution de la viabilité cellulaire au bout de 2 jours et l'absence de croissance cellulaire tout au long de l'étude (Fig. 1).

En conclusion, il ressort que le milieu de croissance des fibroblastes mis en oeuvre selon l'invention permet la survie et stimule la croissance de fibroblastes humains normaux en l'absence de facteurs de croissance exogènes.

### c) Aide à la réparation des composants du derme

### Protocole

- Fragments de peau prélevés sur 8 donneurs, déposés dans des inserts et placés en milieu de culture.
- Irradiation UVA à faibles doses à J0 et J2 (réduction du métabolisme des fibroblastes, altération des macromolécules du tissu conjonctif).
- Milieu selon l'invention ajouté en surface de la peau de J3 à J14 (papier imbibé).
- Témoins négatifs sans irradiation (témoins sains) ou sans application du milieu selon l'invention (UV seuls)
- Analyse histologique (marquage):
   ▪ Collagène (Rouge Sirius) et fibres élastiques (Catéchine) > % de surface occupé au niveau du derme superficiel et moyen (analyse d'image assistée par ordinateur).
   ▪ Glycosaminoglycanes (GAGs) (coloration de Hale) > score semiquantitatif (intensité du marquage).
- Dosage biochimique (spectrocolorimétrique):
   ▪ Collagène total: sur fragments de peaux après digestion enzymatique et broyage.
   ▪ Elastine soluble: dans les surnageants de culture.
   ▪ GAGs: sur fragments de peaux broyés.

### Résultats

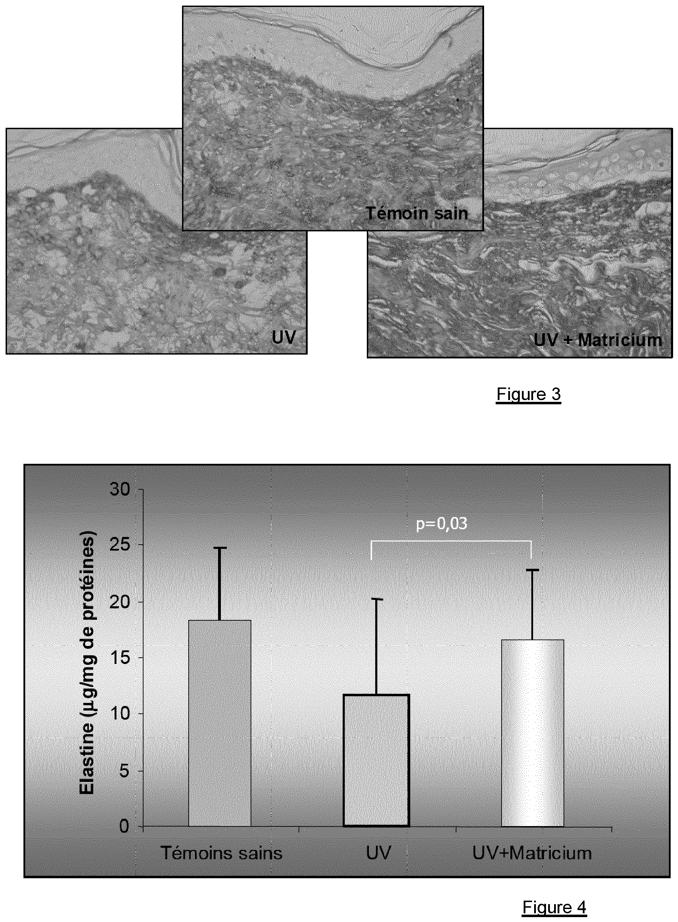

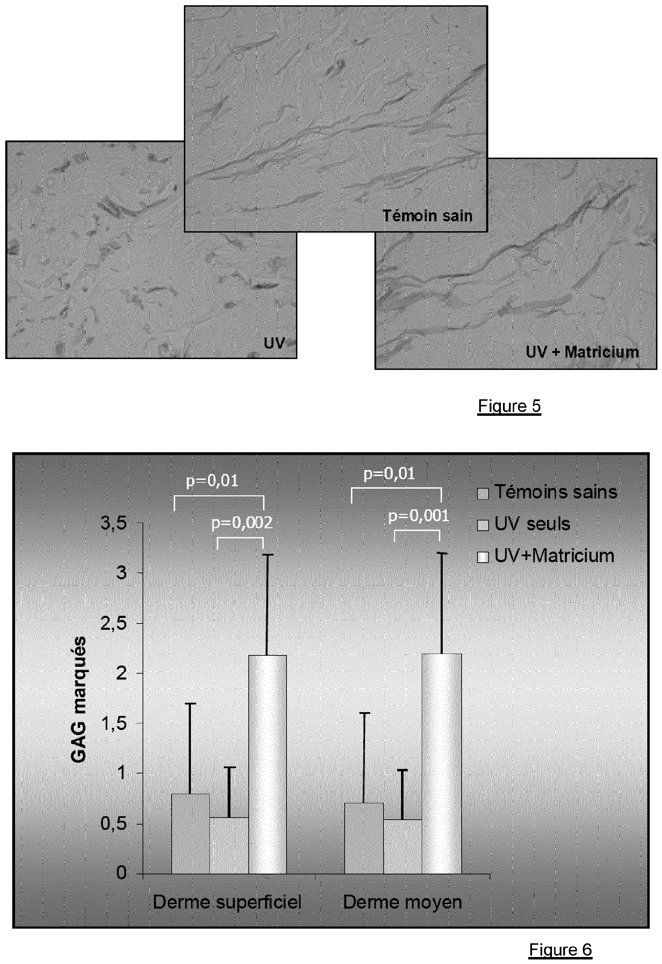

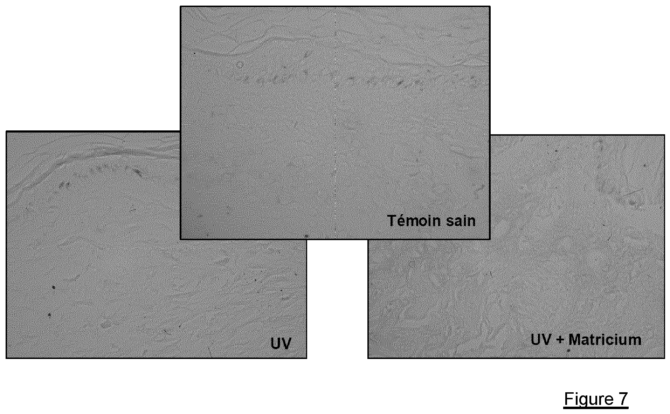

- Réduction significative de la quantité de collagène et d'élastine dans le derme superficiel et profond suite à l'irradiation UV (Fig. 2 à 5).
- Augmentation statistiquement significative du marquage du collagène et de l'élastine après traitement par le milieu selon l'invention (réparation des fibres) (Fig. 2 à 5).
- Stimulation du métabolisme des fibroblastes par le milieu selon l'invention avec augmentation significative du dosage de l'élastine et tendance à l'augmentation du taux de collagène (Fig. 2 à 5).
- Augmentation significative et importante de la quantité de GAGs totaux dans le derme suite à l'application du milieu selon l'invention (Fig. 6 et 7).
- Ce modèle a permis de quantifier sur coupes histologiques les propriétés de réparation du tissu conjonctif du milieu de croissance des fibroblastes mis en oeuvre.

En conclusion, il ressort que ce milieu stimule la réparation et la restauration de composants essentiels du derme (fibres de collagène, d'élastine, GAGs) lors d'altérations tissulaires.

### 2/Préparation d'un gel injectable pour le traitement des rides:

- Milieu de croissance des fibroblastes.
- HA ajouté jusqu'à 3% en poids de la composition totale, avec de manière préférentielle : 0,8% pour le traitement des rides superficielles, 1,6% pour le traitement des rides moyennes et 2% pour le traitement des rides profondes.
- Formulation d'un gel : l'HA est mis en solution dans le milieu de culture des fibroblastes. La concentration en HA détermine la viscosité de la préparation finale. A titre d'exemple, l'HA utilisé est du hyaluronate de sodium dont le poids moléculaire est compris entre 1,3 et 1,8 MDa. Le gel injectable selon l'invention ne comporte aucun additif, l'ensemble des composants de la formule jouant à la fois le rôle d'excipients et d'ingrédients actifs.
- Stérilisation : par filtration sur membrane à 0,22 µm pour le milieu de croissance des fibroblastes, et par stérilisation séparée selon un processus connu de l'homme de l'art pour l'HA.
- Protocole d'injection : Selon la zone à traiter et la profondeur des rides une ou plusieurs séances sont envisagées. Pour maintenir les résultats, il peut être nécessaire de pratiquer des retouches biannuelles, le comblement des rides durant d'autant plus longtemps que la peau est plus jeune.

## Revendications

1. Composition injectable par voie sous-cutanée ou intradermique constituée de :
l'acide hyaluronique ou un de ses sels en tant qu'agent de comblement, l'acide hyaluronique étant sous forme non réticulée; et
un milieu de croissance des fibroblastes exempt de tout facteur de croissance cellulaire ou de tout extrait biologique d'origine animale ou cellulaire, non tracés et/ou de composition indéterminée, ledit milieu de croissance des fibroblastes comprenant :
- des constituants d'acides nucléiques,
- des acides aminés,
- des sucres simples et complexes,
- des vitamines,
- et une fraction inorganique contenant des oligoéléments et des sels minéraux.

2. Composition selon la revendication 1, ***caractérisée* en ce que** l'acide hyaluronique représente de 0,07 à 3% en poids de la composition.

3. Composition selon la revendication 1 ou 2, ***caractérisée* en ce que** le milieu de croissance des fibroblastes comprend en outre un mélange de peptides de lait (MPC).

4. Composition selon l'une des revendications précédentes, ***caractérisée* en ce qu'**elle se présente sous la forme d'un gel.

5. Kit se présentant sous la forme de seringues contenant une composition selon l'une des revendications 1 à 4.

6. Composition selon l'une des revendications 1 à 4 pour son utilisation dans le comblement des dépressions cutanées ou des cicatrices.

7. Procédé cosmétique de comblement des rides et/ou ridules comprenant l'injection de la composition selon l'une des revendications 1 à 4.

8. Composition selon l'une des revendications 1 à 4 comme produit de combinaison pour une utilisation simultanée, séparée ou étalée dans le temps, pour la prévention ou le traitement des rides, ridules, dépressions cutanée et/ou cicatrices.

## Patentansprüche

1. Subkutan oder intradermal injizierbare Zusammensetzung bestehend aus:
Hyaluronsäure oder einem ihrer Salze als Füllmittel, wobei die Hyaluronsäure nicht vernetzt ist; und
ein Fibroblastenwachstumsmedium, das frei von allen Zellwachstumsfaktoren oder allen biologischen Extrakten tierischen oder zellulären Ursprungs ist, die nicht gekennzeichnet und/ oder unbestimmter Zusammensetzung sind, wobei dieses Fibroblastenwachstumsmedium umfasst:
- Nukleinsäure-Bestandteile,
- Aminosäuren,
- einfache und komplexe Zucker,
- Vitamine,
- und einen anorganischen Anteil, der Oligoelemente und mineralische Salze enthält.

2. Zusammensetzung gemäß Anspruch 1, ***dadurch gekennzeichnet,* dass** die Hyaluronsäure zwischen 0,07 und 3 Gewichts-% der Zusammensetzung darstellt.

3. Zusammensetzung gemäß Anspruch 1 oder 2, ***dadurch gekennzeichnet,* dass** das Fibroblastenwachstumsmedium außerdem ein Gemisch aus Milchpeptiden (MPC) enthält.

4. Zusammensetzung gemäß einem der vorstehenden Ansprüche, ***dadurch gekennzeichnet,* dass** sie die Form eines Gels hat.

5. Set in Form von Spritzen, die eine Zusammensetzung gemäß einem der Ansprüche 1 bis 4 enthalten.

6. Zusammensetzung gemäß einem der Ansprüche 1 bis 4, zur Verwendung beim Auffüllen von Hautvertiefungen oder Narben.

7. Kosmetisches Verfahren zum Auffüllen von Falten und/ oder Fältchen, das die Injektion der Zusammensetzung gemäß einem der Ansprüche 1 bis 4 umfasst.

8. Zusammensetzung gemäß einem der Ansprüche 1 bis 4 als Kombinationsprodukt für eine gleichzeitige, getrennte oder zeitlich gestaffelte Verwendung zur Vorbeugung oder der Behandlung von Falten und Fältchen, Hautvertiefungen und/oder Narben.

## Claims

1. Composition for subcutaneous or intradermal injection consisting of:
hyaluronic acid or one of its salts as a filling agent, hyaluronic acid being non crosslinked; and
a fibroblast growth medium free of any cell growth factor or any biological extract of animal or cellular origin, which has not been traced and/or is of undetermined composition, said fibroblast growth medium comprising:
- components of nucleic acids,
- amino acids,
- simple and complex sugars,
- vitamins,
- and an inorganic fraction containing trace elements and mineral salts.

2. Composition according to claim 1, ***characterized* in that** hyaluronic acid makes up 0.07 to 3% by weight of the composition.

3. Composition according to claim 1 or 2, ***characterized* in that** the fibroblast growth medium further comprises a mixture of milk peptides (MPC).

4. Composition according to one of the previous claims, ***characterized* in that** it is in the form of a gel.

5. Kit in the form of syringes containing a composition according to one of claims 1 to 4.

6. Composition according to one of claims 1 to 4 for its use in filling skin depressions or scars.

7. Cosmetic process for filling wrinkles and/or fine lines including the injection of the composition according to one of claims 1 to 4.

8. Composition according to one of claims 1 to 4 as a combination product for simultaneous or separate use or use spread over time for the prevention or treatment of wrinkles, fine lines, skin depressions and/or scars.
